# EUROPÄISCHE PATENTSCHRIFT

(11) **EP 0 662 955 B1**
(45) Veröffentlichungstag und Bekanntmachung des Hinweises auf die Patenterteilung: **09.04.1997**
(21) Anmeldenummer: 93920828.6
(22) Anmeldetag: 27.09.1993
(51) Int. Cl.: C07C 409/04, C07C 407/00

(54) **VERFAHREN ZUM HERSTELLEN VON WASSERARMEN ODER WASSERFREIEN LÖSUNGEN VON ALKYLHYDROPEROXIDEN**
METHOD OF PREPARING ALKYL HYDROPEROXIDE SOLUTIONS CONTAINING LITTLE OR NO WATER
PROCEDE DE PREPARATION DE SOLUTIONS D'HYDROPEROXIDES D'ALKYLE PAUVRES EN EAU OU ANHYDRES

(30) Priorität: 28.09.1992 DE 4232500
(43) Veröffentlichungstag der Anmeldung: 19.07.1995
(73) Patentinhaber: Peroxid-Chemie GmbH, D-82049 Pullach (DE)
(72) Erfinder: APPEL, Hans, D-82049 Höllriegelskreuth (DE); DIEM, Fritz, D-81476 München (DE)
(74) Vertreter: Böhm, Brigitte, Dipl.-Chem. Dr.
(86) Internationale Anmeldenummer: EP9302620
(87) Internationale Veröffentlichungsnummer: WO9407854

(56) Entgegenhaltungen:
- DE-A- 2 855 860
- US-A- 3 778 382
- CHEMICAL ABSTRACTS, vol. 099, no. 17, 24. Oktober 1983, Columbus, Ohio, US; abstract no. 139420, HILL J G ET AL 'Anhydrous tert-butyl hydroperoxide in toluene: the preferred reagent for applications requiring dry TBHP' in der Anmeldung erwähnt & J. ORG. CHEM. (JOCEAH,00223263);83;VOL.48 (20);PP.3607-8 MASSACHUSETTS INST. TECHNOL.;DEP. CHEM.;CAMBRIDGE;02139;MA;USA (US)
- CHEMICAL ABSTRACTS, vol. 099, no. 17, 24. Oktober 1983, Columbus, Ohio, US; abstract no. 139420, HILL J G ET AL 'Anhydrous tert-butyl hydroperoxide in toluene: the preferred reagent for applications requiring dry TBHP'

## Beschreibung

Die Erfindung betrifft ein Verfahren zum Herstellen von wasserfreien oder wasserarmen von tert.-Alkylhydroperoxiden in inerten organischen Lösungsmitteln durch Entwässerung mit H₂SO₄.

Bedingt durch ihre Herstellung sind niedermolekulare Alkylhydroperoxide lediglich mit einem relativ hohen Gehalt an Wasser verfügbar. Außerdem wird Wasser als ein besonders billiges und geeignetes Phlegmatisierungsmittel beim Transport und bei der Lagerung zugesetzt, um so eine sichere Handhabung dieser zur explosionsartigen Zersetzung neigenden Hydroperoxidreagentien zu gewährleisten. So ist z. B. das technisch besonders bedeutsame tert.-Butylhydroperoxid (TBHP) lediglich als TBHP-70 (70 % TBHP und 30 % Wasser) und als TBHP-80 (80 % TBHP, ca. 8 % di-tert.-Butylperoxid und 12 % Wasser) für den Transport zugelassen. Obwohl sich der Wassergehalt solcher Reagentien für eine Reihe von Anwendungen, wie beispielsweise als Initiatoren in der Emulsionspolymerisation, nicht störend auswirkt, hat es sich jedoch gezeigt, daß ein nennenswerter Gehalt an Wasser bei anderen organischen Synthesen, wie beispielsweise bei der asymmetrischen Epoxidation, störend ist und diese in manchen Fällen sogar verhindert. Es besteht daher schon seit langem das Bemühen, konzentrierte, wasserfreie Lösungen von Alkylhydroperoxiden bereitzustellen.

So wurde bereits von P. D. Bartlett et al., J. Am. Chem. Soc. 80, 1398 (1958) ein Verfahren vorgeschlagen, welches später von Böck, Dissertation Univ. München, 1968, verbessert worden ist, tert.-Butylhydroperoxid (TBHP) mittels einer azeotropen Vakuumdestillation zu entwässern. In diesem Verfahren muß TBHP auf ca. 50 bis 70°C erwärmt werden, was mit einem hohen Explosionsrisiko verbunden ist. Daher ist diese Vorgehensweise zur Anwendung in technischem Maßstab nicht geeignet.

J. G. Hill et al. beschreibt in J. Org. Chem. 1983, 48, 3607-3608 die Verwendung von Benzol und Toluol zum azeotropen Entfernen von Wasser. Dabei wird die wäßrige TBHP-Lösung mit Benzol oder Toluol versetzt und das Wasser unter Rückfluß abdestilliert, bis eine konstante Sumpftemperatur die vollständige Entfernung des Wassers anzeigt. Dieses Verfahren hat jedoch ebenfalls den Nachteil, daß auf recht beachtliche Temperaturen, nämlich auf bis zu 107°C erhitzt werden muß. Damit ist jedoch auch dieses Verfahren wegen der thermischen Beanspruchung in einer geschlossenen Destillationsappartur aufgrund der die Sicherheit beeinträchtigenden Probleme nicht für den industriellen Maßstab geeignet. Darüber hinaus sind auch die mit diesem Verfahren erhaltenen Konzentrationen zu gering.

H. Langhals et al., Chem. Ber. 113, 3662-3665 (1980) beschreibt die Verwendung von Molekularsieben zum Trocknen von TBHP und führt ausdrücklich an, daß sich saure Trocknungsmittel wie konzentrierte Schwefelsäure oder Phosphorpentoxid nicht zur Entwässerung von wasserhaltigem TBHP eignen, da diese zur Zersetzung der Peroxide führen sollen. Das von Langhals et al. vorgeschlagene Verfahren zur mehrmaligen Trocknung mit Molekularsieben ist jedoch sehr material- und zeitaufwendig und daher ebenfalls nicht für die Herstellung im technischen Maßstab geeignet. Darüber hinaus muß das verwendete Molekularsieb frei von Schwermetallen sein und es muß vor Gebrauch aufwendig behandelt werden. Zur Trocknung selbst muß gemäß dem angegebenen Beispiel das TBHP fünfmal bei -4°C 3 Stunden lang behandelt werden, um eine ausreichende Wasserentfernung zu erreichen.

Die Erfindung hat daher zum Ziel, ein einfaches und im technischen Maßstab mit leicht handhabbaren handelsüblichen Chemikalien durchzuführendes Trocknungsverfahren für tert.- Alkylhydroperoxide bereitzustellen.

Diese Aufgabe wird erfindungsgemäß durch ein Verfahren zum Herstellen zum wasserfreien oder wasserarmen Lösungen von Alkylhydroperoxiden in inerten Lösungsmitteln durch Entwässern mit H₂SO₄ gelöst, welches dadurch gekennzeichnet ist, daß man daß man zu einer wäßrigen Lösung des tert.-Alkylhydroperoxids ein oder mehrere inerte organische Lösungsmittel sowie Schwefelsäure in einer solchen Menge und Konzentration zusetzt, daß in der nach Vermischen und anschließender Separation der Phasen entstehenden wäßrigen Phase sich mindestens 49 % H₂SO₄ befinden und der Wassergehalt in der organischen Phase höchstens 2,5 % beträgt. Auf diese Weise läßt sich auf einfache Weise eine befriedigende Entwässerung der Alkylhydroperoxidlösungen erreichen.

Vorzugsweise führt man das erfindungsgemäße Verfahren in solcher Weise durch, daß man
- in einem ersten Entwässerungsschritt durch Zusetzen von einem oder mehreren inerten organischen Lösungsmitteln und einer geringen Menge an Schwefelsäure zu einer wäßrigen Lösung des tert.-Alkyl-hydroperoxids eine aus einer wäßrigen und einer organischen Phase bestehende Mischung bildet, nach Separierung der Phasen die organische Phase von der wäßrigen Phase auf an sich bekannte Weise abtrennt, und
- zur organischen Phase in mindestens einem weiteren Entwässerungsschritt noch soviel H₂SO₄ zusetzt, wie nötig ist, um den Wassergehalt in der organischen Phase auf höchstens 2,5 % zu verringern, wobei man die Säuremenge so wählt, daß im letzten Entwässerungsschritt sich mindestens 49 % H₂SO₄ in der wäßrigen Phase befindet, gegebenenfalls auch noch eine weitere Menge des oder der organischen Lösungsmittel zusetzt und wiederum die wäßrige Phase auf an sich bekannte Weise abtrennt.

Es wurde nämlich überraschenderweise festgestellt, daß sich Alkylhydroperoxide besonders vorteilhaft mit mehr oder weniger konzentrierter Schwefelsäure ohne signifikante Zersetzung entwässern lassen, wenn man eine wäßrige Alkylhydroperoxidlösung mit einem inerten organischen Lösungsmittel mischt und zuerst nur eine geringe Menge der zur Wasserentfernung benötigten Schwefelsäure zusetzt. Dabei wird mindestens soviel H₂SO₄ zugesetzt, daß sich eine wäßrige und eine organische Phase bildet. Nach Abtrennen der wäßrigen Phase kann dann in mindestens einem weiteren Entwässerungsschritt soviel H₂SO₄ zugesetzt werden, wie benötigt wird, um aus der organischen Phase das Wasser auf das jeweils gewünschte Maß, nämlich höchstens 2,5 Restwasser, wenn gemessen nach der Methode von Karl Fischer (Angew.Chemie 47 (1935), 394), abzuscheiden. Die hierbei jeweils erhaltene wäßrige Phase wird wiederum auf eine an sich bekannte Weise abgetrennt. Die benötigte Gesamtmenge an Schwefelsäure läßt sich durch einfache, dem Fachmann bekannte Maßnahmen leicht bestimmen oder ist durch einfache Versuche schnell zu ermitteln. In der letzten Stufe sollte die Schwefelsäurekonzentration mindestens 49 % betragen, höhere Werte, bis ca. 53 %, sind ebenfalls vorteilhaft. Darüber muß anhand der eventuellen Sicherheitsrisiken abgecheckt werden, welche Konzentrationen noch sinnvoll sind.

Besonders geeignet ist das erfindungsgemäße Verfahren für niedere, insbesondere tertiäre C₄-C₆-Alkylhydroperoxide, wobei insbesondere tert.-Butylhydroperoxid und tert.-Amylhydroperoxid bevorzugt sind.

In dem erfindungsgemäßen Verfahren wird zweckmäßigerweise eine mindestens 50 %-ige H₂SO₄ und vorzugsweise eine ca. 72 %-ige H₂SO₄ verwendet. Auch eine handelsübliche konzentrierte Schwefelsäure oder auch Oleum ist im erfindungsgemäßen Verfahren verwendbar, wobei jedoch aus Gründen der Effektivität einerseits und aufgrund von Sicherheitsaspekten andererseits die ca. 72 %ige H₂SO₄ sich als besonders geeignet erwiesen hat.

Im erfindungsgemäßen Verfahren hat es sich auch als zweckmäßig erwiesen, im ersten Entwässerungsschritt 0,05 - 15 g H₂SO₄, und vorzugsweise 0,5 - 5 g H₂SO₄ pro 100 g Wasser zuzusetzen. Besonders bevorzugt ist es, 0,1 - 10 g an Schwefelsäure pro 100 g in der Mischung des ersten Entwässerungsschrittes vorliegenden Wassers zuzusetzen.

In dem weiteren Entwässerungsschritt hat es sich als vorteilhaft erwiesen, 5 - 110 g H₂SO₄ pro 100 g ursprünglich vorhandenem H₂O zuzusetzen. Besonders zweckmäßig sind 35 - 75 g, wobei jedoch 40 - 60 g ganz besonders bevorzugt sind.

In einer besonders bevorzugten erfindungsgemäßen Ausführungsform werden drei Entwässerungsschritte durchgeführt und dabei im dritten Schritt noch 5 - 30 g H₂SO₄, und vorzugsweise 8 - 20 g H₂SO₄ pro 100 g des ursprünglich vorhandenen Wassers eingesetzt, um den Wassergehalt der Alkylhydroperoxidlösung noch weiter zu verringern. Ganz besonders ist es bevorzugt, in der dritten Entwässerungsstufe 10 - 15 g H₂SO₄ pro 100 g des ursprünglich vorhandenen Wassers einzusetzen.

Wird in dem erfindungsgemäßen Verfahren eine Schwefelsäure verwendet, die eine Konzentration von weniger als 100 % aufweist, so muß in allen Entwässerungsstufen der Wassergehalt dieser Schwefelsäure zur Menge des ursprünglich vorhandenen Wassers hinzugerechnet werden.

Wird eine noch weiter gehende Trocknung gewünscht, als dies mit H₂SO₄ möglich ist, so hat es sich als zweckmäßig erwiesen, den verbleibenden minimalen Restgehalt an Wasser mittels Molekularsieben zu entfernen und so eine praktisch vollständig entwässerte bzw. trockene tert.-Alkylhydroperoxidlösung zu erhalten. Es hat sich auch als zweckmäßig erwiesen, die den erfindungsgemäß entwässerten Lösungen noch anhaftende Schwefelsäure mittels einer Neutralisation zu entfernen. Dabei wird üblicherweise mit einer festen Base neutralisiert.

Die feste Base wird dann von der neutralisierten Lösung mittels an sich bekannter Verfahren abgetrennt. Zweckmäßige Verfahren sind Filtration, Dekantieren oder Zentrifugieren der Lösung. Besonders geeignete Basen sind Carbonate und Bicarbonate, wobei die Alkali- und Erdalkalisalze besonders geeignet sind. Als ganz besonders geeignet haben sich Natrium- und Kaliumcarbonate und -bicarbonate erwiesen, wovon wiederum die Natriumsalze bevorzugt sind.

Im erfindungsgemäßen Verfahren können alle Lösungsmittel verwendet werden, die gegenüber den tert.-Alkylhydroperoxiden und ggfs. gegenüber eventuellen Reaktionspartnern inert sind und/oder phlegmatisch wirken. Für das erfindungsgemäße Verfahren eignen sich daher aliphatische, cycloaliphatische und aromatische Kohlenwasserstoffe und insbesondere deren Halogenderivate. Als ganz besonders geeignet haben sich für das erfindungsgemäße Verfahren solche Lösungsmittel erwiesen, die einen Siedepunkt aufweisen, der oberhalb 150°C liegt. Als ganz besonders bevorzugtes, phlegmatisierendes Lösungsmittel wird im erfindungsgemäßen Verfahren Isododecan verwendet. In der Praxis hat sich sich aufgrund der Reaktivität der Hydroperoxide als zweckmäßig herausgestellt, die Konzentration der Hydroperoxide so einzustellen, daß der tert.-Alkylhydroperoxid-Gehalt im inerten Lösungsmittel 75 % und vorzugsweise 65 % nicht überschreitet. Im allgemeinen wird soviel Lösungsmittel zugesetzt, daß die Lösungen einen Gehalt an 20 - 75 % bzw. 40 - 65 % Peroxid aufweisen. Höhere Gehalte sind jedoch keineswegs ausgeschlossen.

Im erfindungsgemäßen Verfahren hat es sich auch als zweckmäßig erwiesen, im ersten Entwässerungsschritt nur einen Teil des Lösungsmittels und zwar höchstens bis zu 80 %, üblicherweise 20 - 80 %, und vorzugsweise 30 - 50 % zuzusetzen. Das restliche Lösungsmittel wird dann in den nächsten Entwässerungsstufen entweder teilweise oder auch auf einmal zugesetzt. Als besonders zweckmäßig hat sich die Zugabe des restlichen Lösungsmittels in dem zweiten Entwässerungsschritt erwiesen.

Im erfindungsgemäßen Verfahren hat es sich als vorteilhaft erwiesen, die abgetrennte wäßrige Schwefelsäurelösung nach ihrem Abtrennen mit dem inerten Lösungsmittel zu extrahieren. Dieses inerte Lösungsmittel kann dann im erfindungsgemäßen Verfahren zur Entwässerung verwendet werden, wodurch sich die Ausbeute erhöhen läßt. Auch die wasserhaltige Schwefelsäurelösung wird zweckmäßigerweise im erfindungsgemäßen Verfahren für neue Wasserabmischungen recyclisiert. Dabei ist es möglich, die in späteren Entwässerungsschritten anfallende Schwefelsäure zur Wasserentfernung in der ersten Entwässerungsstufe zu verwenden. Es ist aber auch möglich, die wasserhaltige Schwefelsäure durch Versetzen mit konzentrierter H₂SO₄ oder Oleum auf die jeweils im gleichen oder einem anderen Entwässerungsschritt benötigte Konzentration einzustellen.

Das erfindungsgemäße Verfahren erlaubt eine praktisch vollständige Entwässerung von tert.-Alkylhydroperoxiden, benötigt zu seiner Durchführung keine Energiezufuhr und ist mittels gängiger einfacher und anspruchsloser apparativer Vorrichtungen ausführbar. Darüber hinaus werden nur geringe Mengen an handelsüblichen Chemikalien benötigt, die zudem in den Herstellungsprozeß für die entsprechenden Hydroperoxide (Alkohol + H₂O₂/H₂SO₄) rückführbar sind.

Die folgenden Beispiele sollen die Erfindung näher erläutern.

### Beispiel

Es wurden 900 g TBHP-70 (mit einem Wassergehalt von 270 g H₂O), 80 g Isododecan und 2 ml einer 72 %-igen H₂SO₄ versetzt und nach Einstellung des Phasengleichgewichts wurde in jeder Phase die Konzentration an Wasser, H₂SO₄ und Hydroperoxid bestimmt. Die Ergebnisse in der folgenden Tabelle aufgeführt.

Dann wurde nach Abtrennung der wäßrigen Phase in einem zweiten Entwässerungsschritt nochmals 200 g Isododecan und 105 ml der 72 %-igen H₂SO₄ zugesetzt und nach Einstellung des Phasengleichgewichts wurde in jeder Phase die Konzentration, wie zuvor angegegeben, bestimmt. Nach Abtrennung der wäßrigen Phase wurden nochmal 30 ml an 72 %-iger H₂SO₄ zugesetzt und wie zuvor der Gehalt an Reagentien in jeder Phase bestimmt. Die Ergebnisse sind ebenfalls der folgenden Tabelle zu entnehmen.

Nach Neutralisation mit 12 g festem Natriumcarbonat und Filtration wurden 860 g Lösung mit einem TBHP-Gehalt von 67 % erhalten, was einer Ausbeute von 91 % der eingesetzten Menge entspricht.

Die Bestimmung des Wassergehalts in der organischen Phase erfolgte nach der Methode von Karl Fischer (Karl-Fischer Reagenz der Firma Riedel de Haën).

**Tabelle**

| Extraktionsstufe | 1 | 2 | 3 |
|---|---|---|---|
| Eingesetzt: | | | |
| TBHP-70 | 900 g | | |
| Isododecan | 80 g | 200g | |
| H₂SO₄, 72 % | 2 ml | 105 ml | 30 ml |
| | | | |

| Organ. Phase | | | |
|---|---|---|---|
| Wasser, %* | 19,0 | 3,5 | 1,7 |
| H₂SO₄, % | 0,05 | 0,1 | 0,25 |
| TBHP, % x) | 70-72 | 64-66 | 65-67 |
| | | | |

| Wäßrige Phase | | | |
|---|---|---|---|
| TBHP, % | 14,8 | 3,6 | 4,0 |
| H₂SO₄, % | 1,6 | 39,0 | 52,0 |

| | | | |
|---|---|---|---|
| x) Grenzwerte aus mehreren Versuchen | | | |
| * Bei der Bestimmung mit dem Karl-Fischer-Reagenz werden auch bei mit Hilfe von Molekularsieben vollständig entwässerten Lösungen noch Werte von ca. 0,6 % Wasser erhalten. Dieser Grundwert ist methodebedingt, weshalb die Wassergehaltswerte tatsächlich um etwa 0,6 % niedriger liegen als angegeben. | | | |

Nach Neutralisation mit 12 g festem Natriumcarbonat und Filtration wurden ca. 860 g Lösung mit einem TBHP-Gehalt von 66 - 67 % erhalten, entsprechend 91 % der Einsatzmenge.

## Patentansprüche

1. Verfahren zum Herstellen von wasserarmen oder wasserfreien Lösungen von tert.-Alkylhydroperoxiden in inerten Lösungsmitteln durch Entwässerung mit H₂SO₄,
**dadurch gekennzeichnet**, daß man zu einer wäßrigen Lösung des tert.-Alkylhydroperoxids ein oder mehrere inerte organische Lösungsmittel sowie Schwefelsäure in einer solchen Menge und Konzentration zusetzt, daß in der nach Vermischen und anschließender Separation der Phasen entstehenden wäßrigen Phase sich mindestens 49 % H₂SO₄ befinden und der Wassergehalt in der organischen Phase höchstens 2,5 % beträgt.

2. Verfahren nach Anspruch 1,
**dadurch gekennzeichnet,** daß man
- in einem ersten Entwässerungsschritt durch Zusetzen von einem oder mehreren inerten organischen Lösungsmitteln und einer geringen Menge an Schwefelsäure zu einer wäßrigen Lösung des tert.-Alkylhydroperoxids eine aus einer wäßrigen und einer organischen Phase bestehende Mischung bildet, nach Separierung der Phasen die organische Phase von der wäßrigen Phase auf an sich bekannte Weise abtrennt, und
- zur organischen Phase in mindestens einem weiteren Entwässerungsschritt noch soviel H₂SO₄ zusetzt, wie nötig ist, um den Wassergehalt in der organischen Phase auf höchstens 2,5 % zu verringern, wobei man die Säuremenge so wählt, daß im letzten Entwässerungsschritt sich mindestens 49 % H₂SO₄ in der wäßrigen Phase befindet, gegebenenfalls auch noch eine weitere Menge des oder der organischen Lösungsmittel zusetzt und wiederum die wäßrige Phase auf an sich bekannte Weise abtrennt.

3. Verfahren nach Anspruch 1 oder 2,
**dadurch gekennzeichnet,** daß man eine mindestens 15 %-ige und insbesondere eine mindestens 50 %ige Schwefelsäure verwendet.

4. Verfahren nach Anspruch 1, 2 oder 3,
**dadurch gekennzeichnet,** daß man eine 72 %-ige Schwefelsäure verwendet.

5. Verfahren nach einem der vorhergehenden Ansprüche,
**dadurch gekennzeichnet**, daß man die erhaltene wasserfreie oder wasserarme Alkylhydroperoxidlösung mit einer festen Base neutralisiert.

6. Verfahren nach Anspruch 5,
**dadurch gekennzeichnet**, daß man mit einem Carbonat und/oder Bicarbonat neutralisiert.

7. Verfahren nach Anspruch 6,
**dadurch gekennzeichnet**, daß das Carbonat oder Bicarbonat ein Alkali- und/oder Erdalkalisalz ist.

8. Verfahren nach Anspruch 7,
**dadurch gekennzeichnet**, daß das Alkalisalz ein Kalium- und/oder Natriumsalz ist.

9. Verfahren nach einem der vorhergehenden Ansprüche,
**dadurch gekennzeichnet**, daß man im ersten Entwässerungsschritt 0,05 - 15 g H₂SO₄ pro 100 g Wasser zusetzt.

10. Verfahren nach einem der vorhergehenden Ansprüche,
**dadurch gekennzeichnet,** daß man in dem ersten Entwässerungsschritt 0,5 - 5 g H₂SO₄ pro 100 g H₂O zusetzt.

11. Verfahren nach einem der vorhergehenden Ansprüche,
**dadurch gekennzeichnet,** daß man in mindestens einem weiteren Entwässerungsschritt 5 bis 110 g H₂SO₄ pro 100 g ursprünglich vorhandenem H₂O zusetzt.

12. Verfahren nach einem der vorhergehenden Ansprüche,
**dadurch gekennzeichnet,** daß man in mindestens einem weiteren Entwässerungsschritt 35 - 75 g H₂SO₄ pro 100 g ursprünglich vorhandenem H₂O zusetzt.

13. Verfahren nach einem der vorhergehenden Ansprüche,
**dadurch gekennzeichnet,** daß man drei Entwässerungsschritte durchführt und in dem dritten Entwässerungsschritt mit 5 - 30 g H₂SO₄ pro 100 g des ursprünglich vorhandenen Wassers den Wassergehalt noch weiter verringert.

14. Verfahren nach Anspruch 11,
**dadurch gekennzeichnet,** daß man in dem dritten Entwässerungsschritt 8 - 20 g H₂SO₄ pro 100 g des ursprünglich vorhandenen Wassers einsetzt.

15. Verfahren nach einem der vorhergehenden Ansprüche,
**dadurch gekennzeichnet**, daß man das bereits entwässerte Alkylhydroperoxid mit einem Molekularsieb noch weiter trocknet.

16. Verfahren nach einem der vorhergehenden Ansprüche,
**dadurch gekennzeichnet,** daß das inerte, organische Lösungsmittel ein aliphatischer, cycloaliphatischer oder aromatischer Kohlenwasserstoff oder eine Mischung davon ist.

17. Verfahren nach Anspruch 16,
**dadurch gekennzeichnet,** daß das Lösungsmittel einen Siedepunkt aufweist, der größer als 150°C ist.

18. Verfahren nach einem der vorhergehenden Ansprüche,
**dadurch gekennzeichnet,** daß das Lösungsmittel Isododecan ist.

19. Verfahren nach einem der vorhergehenden Ansprüche,
**dadurch gekennzeichnet,** daß das Lösungsmittel ein halogeniertes Lösungsmittel ist.

20. Verfahren nach einem der vorhergehenden Ansprüche,
**dadurch gekennzeichnet,** daß das t-Alkylhydroperoxid ein C₄-C₆-Alkylhydroperoxid ist.

21. Verfahren nach einem der vorhergehenden Ansprüche,
**dadurch gekennzeichnet**, daß in der ersten Stufe höchstens 50% des inerten Lösungsmittels zugesetzt werden.

22. Verfahren nach einem der vorhergehenden Ansprüche,
**dadurch gekennzeichnet**, daß in der ersten Stufe höchstens 30 % des inerten Lösungsmittels zugesetzt wird.

23. Verfahren nach einem der vorhergehenden Ansprüche,
**dadurch gekennzeichnet,** daß man die abgetrennten wäßrigen Phasen mit dem inerten Lösungsmittel extrahiert und dieses für weitere zu entwässernde Alkylhydroperoxide verwendet.

## Claims

1. A method for preparing solutions of tert.-alkyl hydroperoxides in inert solvents with little or no water content by dewatering with H₂SO₄, characterised in that one or more inert organic solvents as well as sulphuric acid are added to an aqueous solution of the tert.-alkyl hydroperoxide, in such a quantity and concentration that in the aqueous phase formed after the mixing and subsequent separation of the phases there is at least 49 % H₂SO₄, and the water content in the organic phase is at most 2.5 %.

2. A method according to Claim 1, characterised in that
- in a first dewatering step, by adding one or more inert organic solvents and a small amount of sulphuric acid to an aqueous solution of the tert.-alkyl hydroperoxide a mixture consisting of an aqueous and an organic phase forms, after separation of the phases, the organic phase is separated from the aqueous phase in a manner known per se, and
- there is added to the organic phase, in at least one additional dewatering step, as much H₂SO₄ as is needed to reduce the water content in the organic phase to at most 2.5 %, the amount of acid being selected such that in the final dewatering step at least 49 % H₂SO₄ is present in the aqueous phase, optionally yet another amount of the organic solvent or solvents is added, and again the aqueous phase is separated in a manner known per se.

3. A method according to Claim 1 or 2, characterised in that an at least 15 % and, in particular, an at least 50 % sulphuric acid is used.

4. A method according to Claim 1, 2 or 3, characterised in that a 72 % sulphuric acid is used.

5. A method according to any one of the preceding Claims, characterised in that the resultant alkyl hydroperoxide solution with little or no water content is neutralised with a solid base.

6. A method according to Claim 5, characterised in that a carbonate and/or bicarbonate is used for neutralisation.

7. A method according to Claim 6, characterised in that the carbonate or bicarbonate is an alkali salt and/or alkaline earth salt.

8. A method according to Claim 7, characterised in that the alkali salt is a potassium and/or sodium salt.

9. A method according to any one of the preceding Claims, characterised in that in the first dewatering step 0.05 to 15 g of H₂SO₄ is added per 100 g of water.

10. A method according to any one of the preceding Claims, characterised in that in the first dewatering step 0.5 to 5 g of H₂SO₄ is added per 100 g of H₂O.

11. A method according to any one of the preceding Claims, characterised in that in at least one further dewatering step 5 to 110 g of H₂SO₄ is added per 100 g of the H₂O originally present.

12. A method according to any one of the preceding Claims, characterised in that in at least one further dewatering step 35 to 75 g of H₂SO₄ are added per 100 g of the H₂O originally present.

13. A method according to any one of the preceding Claims, characterised in that three dewatering steps are carried out and in the third dewatering step with 5 to 30 g H₂SO₄ per 100 g of the water originally present the water content is reduced still further.

14. A method according to Claim 11, characterised in that in the third dewatering step 8 to 20 g of H₂SO₄ are used per 100 g of the water originally present

15. A method according to any one of the preceding Claims, characterised in that the already dewatered alkyl hydroperoxide is dried further with a molecular sieve.

16. A method according to any one of the preceding Claims, characterised in that the inert organic solvent is aliphatic, cycloaliphatic or aromatic hydrocarbon or a mixture thereof.

17. A method according to Claim 16, characterised in that the solvent has a boiling point which is higher than 150°C.

18. A method according to any one of the preceding Claims, characterised in that the solvent is isododecane.

19. A method according to any one of the preceding Claims, characterised in that the solvent is a halogenated solvent.

20. A method according to any one of the preceding Claims, characterised in that the t-alkyl hydroperoxide is a C₄-C₆ alkyl hydroperoxide.

21. A method according to any one of the preceding Claims, characterised in that in the first stage at most 50 % of the inert solvent is added.

22. A method according to any one of the preceding Claims, characterised in that in the first stage at most 30 % of the inert solvent is added.

23. A method according to any one of the preceding Claims, characterised in that the separated aqueous phases are extracted with the insert solvent and the latter is used for additional alkyl hydroperoxide to be dewatered.

## Revendications

1. Procédé de préparation de solutions anhydres ou contenant peu d'eau d'hydroperoxydes de tert-alkyle dans des solvants inertes par déshydratation avec H₂SO₄, caractérisé en ce que l'on ajoute à une solution aqueuse de l'hydroperoxyde de tert-alkyle un ou plusieurs solvants organiques inertes et de l'acide sulfurique en une quantité et une concentration telles que la phase aqueuse formée après le mélange et la séparation subséquente des phases contient au moins 49 % de H₂SO₄ et que la teneur en eau de la phase organique est d'au plus 2,5 %.

2. Procédé selon la revendication 1, caractérisé en ce que
- dans une première étape de déshydratation, on forme un mélange composé d'une phase aqueuse et d'une phase organique en ajoutant un ou plusieurs solvants organiques inertes et une petite quantité d'acide sulfurique à une solution aqueuse de l'hydroperoxyde de tert-alkyle, et, une fois que les phases se sont séparées, on sépare la phase organique de la phase aqueuse de façon connue en soi, et
- dans au moins une autre étape de déshydratation, on rajoute à la phase organique la quantité de H₂SO₄ nécessaire pour réduire la teneur en eau de la phase organique à 2,5 % au plus, en choisissant la quantité d'acide de façon que, dans la dernière étape de déshydratation, il y ait au moins 49 % de H₂SO₄ dans la phase aqueuse, on rajoute éventuellement une nouvelle quantité du ou des solvants organiques et on sépare de nouveau la phase aqueuse de façon connue en soi.

3. Procédé selon la revendication 1 ou 2, caractérisé en ce que l'on utilise un acide sulfurique à au moins 15 % et en particulier un acide sulfurique à au moins 50 %.

4. Procédé selon la revendication 1, 2 ou 3, caractérisé en ce que l'on utilise un acide sulfurique à 72 %.

5. Procédé selon l'une des revendications précédentes, caractérisé en ce que l'on neutralise avec une base solide la solution d'hydroperoxyde anhydre ou contenant peu d'eau obtenue.

6. Procédé selon la revendication 5, caractérisé en ce que l'on neutralise avec un carbonate et/ou un bicarbonate.

7. Procédé selon la revendication 6, caractérisé en ce que le carbonate ou le bicarbonate est un sel de métal alcalin et/ou de métal alcalino-terreux.

8. Procédé selon la revendication 7, caractérisé en ce que le sel de métal alcalin est un sel de potassium et/ou de sodium.

9. Procédé selon l'une des revendications précédentes, caractérisé en ce que l'on ajoute 0,05 à 15 g de H₂SO₄ pour 100 g d'eau dans la première étape de déshydratation.

10. Procédé selon l'une des revendications précédentes, caractérisé en ce que l'on ajoute 0,5 à 5 g de H₂SO₄ pour 100 g de H₂O dans la première étape de déshydratation.

11. Procédé selon l'une des revendications précédentes, caractérisé en ce que, dans au moins une autre étape de déshydratation, on ajoute 5 à 110 g de H₂SO₄ pour 100 g de l'eau présente au départ.

12. Procédé selon l'une des revendications précédentes, caractérisé en ce que, dans au moins une autre étape de déshydratation, on ajoute 35 à 75 g de H₂SO₄ pour 100 g de l'eau présente au départ.

13. Procédé selon l'une des revendications précédentes, caractérisé en ce que l'on effectue 3 étapes de déshydratation et en ce que, dans la troisième étape de déshydratation, on réduit encore la teneur en eau avec 5 à 30 g de H₂SO₄ pour 100 g de l'eau présente au départ.

14. Procédé selon la revendication 11, caractérisé en ce que, dans la troisième étape de déshydratation, on ajoute 8 à 20 g de H₂SO₄ pour 100 g de l'eau présente au départ.

15. Procédé selon l'une des revendications précédentes, caractérisé en ce que l'on sèche de façon encore plus poussée, à l'aide d'un tamis moléculaire, l'hydroperoxyde d'alkyle déjà déshydraté.

16. Procédé selon l'une des revendications précédentes, caractérisé en ce que le solvant organique inerte est un hydrocarbure aliphatique, cycloaliphatique ou aromatique ou un de leurs mélanges.

17. Procédé selon la revendication 16, caractérisé en ce que le solvant a un point d'ébullition supérieur à 150°C.

18. Procédé selon l'une des revendications précédentes, caractérisé en ce que le solvant est l'isododécane.

19. Procédé selon l'une des revendications précédentes, caractérisé en ce que le solvant est un solvant halogéné.

20. Procédé selon l'une des revendications précédentes, caractérisé en ce que l'hydroperoxyde de tert-alkyle est un hydroperoxyde d'alkyle en C₄-C₆.

21. Procédé selon l'une des revendications précédentes, caractérisé en ce que, dans la première étape, on ajoute au plus 50 % du solvant inerte.

22. Procédé selon l'une des revendications précédentes, caractérisé en ce que, dans la première étape, on ajoute au plus 30 % du solvant inerte.

23. Procédé selon l'une des revendications précédentes, caractérisé en ce que l'on extrait les phases aqueuses séparées avec le solvant inerte et on utilise ce dernier pour d'autres hydroperoxydes d'alkyle à déshydrater.
